(19) Europäisches Patentamt
European Patent Office
Office européen des brevets

(11) Publication number: **0 427 711 A1**

(12) **EUROPEAN PATENT APPLICATION**

(21) Application number: **91200001.5**

(51) Int. Cl.[5]: **C07C 41/20**

(22) Date of filing: **14.07.87**

This application was filed on 02.01.1991 as a divisional application to the application mentioned under INID code 60.

(30) Priority: **14.07.86 US 885528**

(43) Date of publication of application: **15.05.91 Bulletin 91/20**

(60) Publication number of the earlier application in accordance with Art.76 EPC: **0 253 637**

(84) Designated Contracting States: **BE DE FR GB IT NL**

(71) Applicant: **EXXON CHEMICAL PATENTS INC.**
**200 Park Avenue**
**Florham Park New Jersey 07932(US)**

(72) Inventor: **Plotkin, Jeffrey Stuart**
**7 Carpenter Court**
**Monsey, New York 10952(US)**
Inventor: **Michaelson, Robert Charles**
**One Glendale Terrace**
**Kinneldon, New Jersey 07405(US)**

(74) Representative: **Mansell, Keith Rodney (GB) et al**
**Exxon Chemical Limited European Patents and Licences Exxon Chemical Technology Centre P O Box 1**
**GB-Abingdon, Oxfordshire OX13 6BB(GB)**

# (54) Hydrogenation of unsaturated ethers.

(57) A process for hydrogenating mixed ethers after separation from hydrocarbon mixture to saturate the unsaturated ether(s) formed from the diolefins present in the hydrocarbon mixture. This may be applied to a process for separating $C^4$ to $C^7$ tertiary mono-olefins(s) present in a $C^4$ to $C^7$ hydrocarbon mixture containing diolefins which comprises selectively reacting said tertiary mono-olefin mixture with a primary alcohol in the presence of an acid catalyst to convert it to tertiary ether(s), separating the tertiary ether(s) from the unreacted hydrocarbon mixture and containing said tertiary ether(s) in the presence of a catalyst to decompose it to tertiary olefin(s) and a primary alcohol.

EP 0 427 711 A1

# HYDROGENATION OF UNSATURATED ETHERS

This invention relates to an improvement in hydrogenating unsaturated ethers such as those produced from diolefins to saturated ethers. Such unsaturated ethers are formed in the presence of saturated ethers in the industrial process for extracting tertiary olefins from streams also containing other olefins and diolefins. In such a process the stream is reacted with methanol to preferentially convert the tertiary olefins to methyl ethers, the methyl ethers are separated by distillation and the separated methyl ether stream is then decomposed over an acid catalyst to recover the pure tertiary olefins. More particularly the invention relates to the improvement of hydrogenating unsaturated ethers found in the separated methyl ether stream prior to decomposition. The invention also relates to preferred catalysts and conditions used for such hydrogenation.

The extraction of iso-olefins is accomplished industrially by exploiting the preferential reactivity of iso-olefins with methanol to form the corresponding methyl ether compounds. After the ethers are isolated by distillation, the pure iso-olefin can be regenerated by decomposition of the ether over acid catalysts at elevated temperature. For example, isobutylene can be extracted from mixed $C_4$ streams by preferential formation of methyl tertiary butyl ether (MTBE), distilling off the remaining light $C_4$ materials, and finally cracking the MTBE back to methanol and isobutylene. Similarly, isoamylene can be extracted from $C_5$ streams by formation of tertiary amyl methyl ether followed by distillation and crackback.

In each of the above examples the presence of diolefins in the mixed streams causes operational problems. Diolefins as well as olefins are produced in steam cracking and/or coking and are often present in small quantities in the mixed stream after removal of the majority of the diolefins in prior processing, e.g., DMF (dimethylformamide) extraction. Such diolefins also readily react with methanol resulting in methyl ether compounds containing one double bond (unsaturated ethers). Methyl ether streams containing even low levels of unsaturated ether will cause rapid fouling of the ether decomposition catalyst. Typically, this problem is solved by selective hydrogenation of the dienes before the stream enters the etherification reactor.

This approach has several drawbacks. Foremost, over-hydrogenation must be avoided. This is not easily accomplished since there are normally very low levels of dienes (1-3%) in the presence of mono-olefins. Overhydrogenation would not only lead to loss of desired mono-olefin product and hydrogen but inasmuch as hydrogenations are exothermic, this situation is a potentially very dangerous one. Additionally, $C_4$ or $C_5$ olefin streams usually contain relatively large amounts of mercaptans. Mercaptans are known noble metal catalyst poisons. All of these factors combined necessitate that a very selective and rugged hydrogenation catalyst be employed. Lastly, treatment of the dienes at this stage requires that the entire olefin containing stream pass through the hydrogenation reactor. This requires the use of a very large and therefore expensive reactor.

## SUMMARY OF THE INVENTION

This invention describes a process for the hydrogenation of unsaturated ethers in the presence of saturated ethers which comprises the steps of:

separating a mixed ether stream containing a minor amount of unsaturated ethers and a major amount of $C_4$ to $C_7$ saturated tertiary ethers from a stream containing $C_4$ to $C_7$ hydrocarbons, and

contacting the mixed ether stream with a hydrogenation catalyst comprising 0.1 to 1.0 weight percent of a Group VIII noble metal dispersed on an alumina having a surface area of 1 to 200 $m^2$/gm, at a temperature of from 40°C to 200°C, in the presence of 1.0 to 2.0 mols of $H_2$/mol of unsaturation present in the mixed ether stream, at a space velocity of 0.5 to 4.0 LHSV to saturate the $C_4$ to $C_7$ unsaturated ethers.

This process may be used in handling the aforementioned operating difficulties arising from the presence of dienes in the olefin stream in the iso-olefin extraction. An application of the process to such an extraction is described in EP-A-253637 from which this application is divided.

## DETAILED DESCRIPTION OF THE INVENTION

The hydrogenation of the present invention is conducted at temperatures of from 40°C to 200°C, preferably 50°C to 180°C, in the presence of from 1.0 to 2.0, preferably 1.0 to 1.3 mols, of hydrogen per mole of unsaturation present in the feed. The reaction is carried out at a spatial velocity, as expressed in

terms of volume of liquid per volume of catalyst per hour (LHSV), of from 0.5 LHSV to 4.0 LHSV, preferably 1.0 LHSV to 3.0 LHSV. The hydrogenation may be carried out in the vapor or liquid phase, preferably in the liquid phase, at pressures of about 200 psig to 500 psig, preferably 250 psig to 350 psig.

Preferably the water content of the feed to the hydrogenation step is kept below 1000 ppm, more preferably below 50 ppm. This is desirable to mitigate the undesirable byproduct formation of ketones and aldehydes. The chemistry of such by product formation is as follows: unsaturated ethers, i.e.:

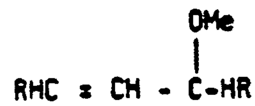

are hydroisomerized over the catalyst to vinyl ethers, i.e.:

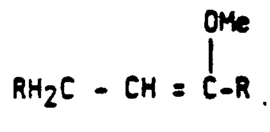

These vinyl ethers are more difficult to hydrogenate than unsaturated ethers and furthermore are easily hydrolyzed to ketones or aldehydes, i.e.:

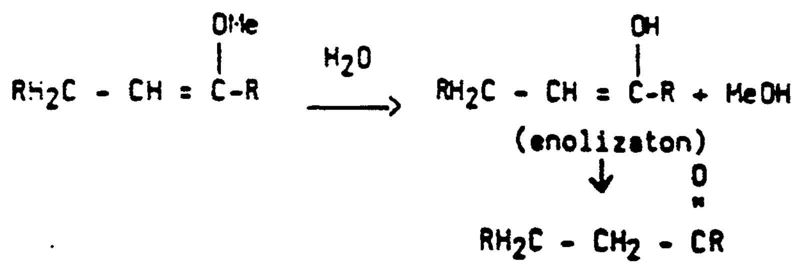


If water is kept out, vinyl ethers can be hydrogenated otherwise they are partially converted to ketones or aldehydes.

The hydrogenation catalyst comprises a noble metal catalyst belonging to group VIII of the periodic table (such as is used conventionally in catalytic hydrogenations) with an alumina carrier material. Preferred catalysts are palladium and platinum. A suitable catalyst which does not belong to said group is in particular Raney-nickel. The catalysts are used in usual amounts, preferably in an amount of from about 0.005 to about 0.2% by weight (metal), calculated on the starting compound to be hydrogenated.

The alumina support has a surface area of 1.0 to 200 $m^2$/gm, preferably 2.0 to 150 $m^2$/g. Preferred aluminas are aluminas of low silica content, preferably below 1% silica content to reduce acidity and isomerization. The amount of metal dispersed on the catalyst is 0.1 to 1.0 weight percent, more preferably 0.1 to 0.5 weight percent.

Particularly preferred catalysts are IFP LD-265 0.3% Pd on alumina catalyst, UCI G-68 0.1% Pd on alumina catalyst and most preferably Calsicat E-144 SDU 0.5% Pd on alumina catalyst.

The mixed ether stream differs from the $C_4$ to $C_7$ hydrocarbon stream in boiling point and, therefore, can be easily separated by conventional methods such as distillation, etc. Monoolefins other than tertiary olefins can be separated in substantially the same manner as the inert saturated hydrocarbons since they are remarkably low in reaction rate with primary alcohol.

For example isobutylene may be converted to either tertiary butyl methyl ether or tertiary butyl ethyl ether, the boiling points of which are 55° C and 73° C, respectively, and are separated easily, since the difference in boiling point between the ether and unreacted $C_4$ mixture used to prepare the ethers is great. Similarly isoamylene may be converted either to tertiary amyl methyl ether or tertiary amyl ethyl ether, the boiling points of which are 86° C and 101° C, respectively, and again these are separated easily due to the difference in boiling points from the unreacted $C_5$ mixture.

After the tertiary ether is separated from the unreacted hydrocarbon mixture, the ether is contacted with the above-mentioned solid hydrogenation catalyst as above described.

Following hydrogenation to saturate the unsaturated ethers present, the hydrogenated product may be

contacted with any of the well-known ether decomposition catalysts to decompose the product back to the tertiary olefin and the primary alcohol. Such catalyst include metal sulfate catalysts, aluminium compounds supported on silica or other carriers, modified cation exchange resin catalysts, etc. Suitable reaction conditions for the decomposition step are temperature of 100-250° C and spatial velocities, as expressed in terms of volume of liquid per volume of catalyst per hour (LHSV) ranging between 0.5 and 30. Conditions are also preferably chosen to maintain conversions of the tertiary ether above 80%.

The present invention will be illustrated in more detail with reference to the following examples, which are not however to be interpreted as limiting the scope of the invention.

A number of preferred hydrogenation catalysts were tested under preferred reaction conditions in the hydrogenation of unsaturated ethers in accordance with the present invention.

The laboratory equipment utilized was a 1/2" O.D. (0.41" ID) 18" long stainless steel reactor. Heat was supplied by circulating hot oil through a jacket surrounding the reactor. Feed was supplied by a high pressure pump and hydrogen was mixed with the feed prior to the reactor inlet and was controlled by a mass flow controller.

The reactor was operated in the upflow mode and the reactor pressure was controlled by a back pressure regulator. In a typical run 30cc of catalyst was used and it was held in place by glass wool plugs.

The reactor effluent was cooled, degassed and collected for analysis by capillary gas chromatagraphy. The gasoeus effluent from the reactor was measured by a wet test meter and the data was used to perform material balance calculations.

Results

The hydrogenations were conducted in the liquid phase over solid palladium on alumina catalysts. The pressure ranges looked at were 200-450 psig while the temperatures ranged from 40-200° C. LHSV's of 0.3 to 4.0 were also looked at. The hydrogen, in most cases, was maintained at 20% molar excess based on the mole percent unsaturation present in the feed.

The TAME feedstock used in all the experiments was produced from an actual isoamylene containing cat cracker stream by reacting it with 50 weight percent methanol at a temperature of 50° C for 6 hours in the presence of an acid catalyst and should be representative of what would be found in a commercial plant with the following exceptions. The TAME does not contain any heavy isoprene ether or ethers of cyclopentadiene. This is because the laboratory distillation of the TAME to separate the unreacted hydrocarbons was so efficient that the heavy isoprene ether and cyclopentadiene ethers went bottoms. To test the hydrogenation of the heavy isoprene ether it was added to the TAME in one of the runs. This is noted in the table below. The sulfur level of the TAME was 10 ppm. The level of sulfur in a commercial feed might be near 50 ppm (40 ppm thiophene, 10 ppm mercaptans). This difference should not change the performance of the catalyst since analysis of the feed and product shows the same level of sulfur. This indicates that the types of compounds containing the sulfur do not have a high affinity for the catalyst. Three different 15 gallon barrels of TAME were used, each containing slightly different amounts of unsaturated ethers. The TAME and unsaturated ether composition of each barrel is given below.

TAME Feed Analysis

| | wt % | | |
|---|---|---|---|
| | Barrel 1 | Barrel 2 | Barrel 3 |
| Component | | | |
| TAME | 96.3 | 92.47 | 94.447 |
| Light Isoprene Ethers | 0.594 | 0.299 | 0.347 |
| Piperylene Ethers | .721 | 2.818 | 2.674 |

IFP LD-265 (Effect of Drying Feed)

The first catalyst tested was IFP LD-265. This catalyst contains 0.3% palladium dispersed on alumina.

The catalyst is manufactured in 2-4 mm spheres; however, to pack the laboratory scale half inch reactor, it was necessary to crush and sieve the spheres to 12-16 mesh. The catalyst as received is in an oxidized form. For use as a hydrogenation catalyst the palladium oxide was first reduced to Pd' by flowing pure hydrogen (100 ml/min) over it at 80 °C for 16 hours.

The results obtained with this catalyst are given in Table 1 below. In the first three runs the pressure was 300 psig and the LHSV was 2, and in run 12560-42015 the pressure was 450 psig and the LHSV 2.5

Table 1

| Exp. No. | Water ppm | T°C | Select to Satd. Pip Ether | Select to Vinyl Ether | Select to 2-Pentanone |
|---|---|---|---|---|---|
| 12560-11-106 | 5000 | 80 | 31.5 | 64.2 | 3.6 |
| 12560-11-106 | 100-200 | 114 | 40.4 | 53.9 | 4.9 |
| 12560-42-6 | 100-200 | 100 | 83.0 | 11.4 | 7.4 |
| 12560-42015 | 100-200 | 100 | 74.5 | 14.4 | 6.7 |

As can be seen when the feed is dry, i.e., 100-200 ppm water, selectivity to desired saturated piperylene ethers is greatly increased and selectivity to undesired vinyl ether is decreased. Selectivity is poor even with dry feeds if the catalyst has been previously exposed to wet feeds which was the case with respect to Run 12560-11-106. In Run 12560-420 15 the feed was spiked with 0.6 weight percent heavy isoprene ether added. The product from this run was analyzed and it was found that 100% of the heavy isoprene ether had been hydrogenated. This shows that both light and heavy unsaturated isoprene ether isomers will smoothly hydrogenate to the expected saturated products.

Calsicat E-144 SDU, 0.5% Pd/alumina, 8 x 14 mesh spheres (#73C-080C) was loaded into the reactor (30 cc/25.05 g). The catalyst was activated by flowing pure hydrogen (100 ml/min) over it at 80 °C for 16 hours. A range of operating temperatures and LHSV's were explored. In all these experimentsthe pressure was kept constant at 300 psig and the hydrogen flow was maintained at 20% molar excess based on the mole percent unsaturation in the TAME feedstock. The screening results are given in Table 2 below.

The effect of temperature upon conversion and selectivity can be seen from the runs shown below. Also, the better performance of Calsicat E-144 SDU as compared to IFP LD-265 and UCI G-68E catalysts screened can also be seen. UCI G-68 catalyst is a 0.1% Pd on alumina selective hydrogenation catalyst with a very low surface area support (2-5 $m^2$/gram).

Table 2

| Run No. | Catalyst | Temperature | Light Isoprene Ether Conv. | Piper. Ether Conv. | Selectivity: Sat. Piper. Ether | Selectivity: Vinyl Ethers | Selectivity: 2-Pentanone | Selectivity: Ketal |
|---|---|---|---|---|---|---|---|---|
| 13203-22 | Calsicat E-144 SDU | 40 | 100 | 93.0 | 87.9 | 14.0 | 2.4 | 0.6 |
| 13203-19 | Calsicat E-144 SDU | 60 | 100 | 93.5 | 95.0 | 2.5 | 2.4 | 0.8 |
| 13203-14 | Calsicat E-144 SDU | 80 | 100 | 92.8 | 95.6 | 2.7 | 2.2 | 0.8 |
| 13203-23 | Calsicat E-144 SDU | 100 | 100 | 94.0 | 97.6 | 0.4 | 2.1 | 0.0 |
| 12560-3 | IFP LD-265 | 80 | 100 | 96.0 | 77.9 | 17.0 | 4.4 | - |
| 12560-1 | UCI G-68C | 80 | 96.0 | 91.4 | 68.2 | 21.7 | 4.3 | - |

Complete conversion of the isoprene ether and complete or very near complete conversion of the piperylene ethers are achieved with Calsicat E-144 SDU at temperatures as low as 40° C. The truly impressive aspect is the very high selectivities (95%) to the desired saturated piperylene ether achieved at only 60° C. At 40° C the selectivity drops to 88% while the vinyl ethers increase to 14%. Apparently, at this mild temperature, the isomerization reaction, which results in vinyl ethers, can effectively compete with the desired hydrogenation reaction.

The superiority of Calsicat E-144 SDU, in terms of selectivity to the saturated piperylene ether over either IFP LD-265 or UCI G-68E, can also been seen from Table 2. Low selectivity to vinyl ethers is also very important because it is these compounds which, when in the presence of even trace amounts of water, will hydrolyze to give 2-pentanone. Although the IFP LD-265 catalyst did give selectivities of saturated piperylene ether as high as 94%, the conditions necessary to accomplish this were relatively much more severe, i.e., 100 °C; LHSV = 1. Calsicat E-144 SDU's enhanced activity will allow operating at temperatures as low as 60 °C while still achieving high selectivity to the saturated piperylene ether.

The ability to operate at low temperature should significantly extend catalyst run length. This is because the rate of catalyst poisoning by sulfur containing compounds is known to be temperature dependent and also starting at low temperatures leaves more room to ramp up the temperature as the catalyst slowly deactivates.

The effect of space velocity upon Calsicat E-144 SDU performance was also investigated. All the runs shown below were at 80 °C and 300 psig.

Table 3

| Run No. | LHSV | Light Isoprene Ether Conv. | Piper. Ether Conv. | Selectivity | | | |
|---|---|---|---|---|---|---|---|
| | | | | Sat. Piper. Ether | Vinyl Ethers | 2-Pentanone | Ketal |
| 13023-11 | 1 | 100 | 94.1 | 98.3 | 0.5 | 0.6 | - |
| 13023-9 | 1.5 | 100 | 93.2 | 99.7 | 0.3 | 2.6 | - |
| 13023-14 | 2 | 100 | 92.8 | 95.6 | 2.7 | 2.2 | 0.8 |

Complete or near complete conversions of both isoprene and piperylene ethers are achieved at LHSV 1-3. Not surprisingly, at the higher space velocities the selectivity to vinyl ethers increases. This is because it is surmised that the vinyl ethers formed are not in contact with the catalyst surface long enough to be totally hydrogenated.

**Claims**

1. A process for the hydrogenation of unsaturated ethers in the presence of saturated ethers which comprises the steps of:
separating a mixed ether stream containing a minor amount of unsaturated ethers and a major amount of $C_4$ to $C_7$ saturated tertiary ethers from a stream containing $C_4$ to $C_7$ hydrocarbons, and
contacting the mixed ether stream with a hydrogenation catalyst comprising 0.1 to 1.0 weight percent of a Group VIII noble metal dispersed on an alumina having a surface area of 1 to 200 $m^2$/gm, at a temperature of from 40°C to 200°C, in the presence of 1.0 to 2.0 mols of $H_2$/mol of unsaturation present in the mixed ether stream, at a space velocity of 0.5 to 4.0 LHSV to saturate the $C_4$ to $C_7$ unsaturated ethers.

2. The process of claim 1 in which the hydrogenation step is carried out in the liquid phase in the presence of a catalyst comprising 0.5 weight percent Pd on alumina having a surface area of approximately 30 to 49 $m^2$/gm.

3. The process of claim 1 or claim 2 in which the hydrogenation catalyst is a 0.5 weight percent Pd on alumina catalyst having a surface area of approximately 35 $m^2$/gm.

4. The process of any of claims 1 to 3 wherein the tertiary ether containing feed to the hydrogenation step contains less than 1000 ppm water, preferably less than 10 ppm water.

European
Patent Office

# EUROPEAN SEARCH REPORT

Application Number

**EP 91 20 0001**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-1 902 264 (MELLE-BEZON)<br>- - - - - | | C 07<br>C 41/20 |
| | | | TECHNICAL FIELDS SEARCHED (Int. Cl.5) |
| | | | C 07 C 41/00 |

The present search report has been drawn up for all claims

| Place of search | Date of completion of search | Examiner |
|---|---|---|
| The Hague | 13 March 91 | VAN GEYT J.J.A. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document